# FASCICULE DE BREVET EUROPEEN

(11) **EP 2 719 424 B1**
(45) Date de publication et mention de la délivrance du brevet: **08.04.2015**
(21) Numéro de dépôt: 13169178.4
(22) Date de dépôt: 24.05.2013
(51) Int. Cl.: A61N 1/362, A61N 1/375, A61N 1/05

(54) **Sonde intraseptale de stimulation du ventricule gauche**
Intraseptale Sonde zur Stimulation des linken Ventrikels
Intraseptal probe for left ventricular stimulation

(30) Priorité: 12.10.2012 FR 1259760
(43) Date de publication de la demande: 16.04.2014
(73) Titulaire: Sorin CRM SAS, 92140 Clamart Cedex (FR)
(72) Inventeur: Ollivier, Jean-François, 91190 Villiers le Bacle (FR)
(74) Mandataire: Dupuis-Latour, Dominique

(56) Documents cités:
- EP-A1- 1 570 880
- EP-A1- 2 246 091
- EP-A1- 2 327 366
- EP-A1- 2 384 784
- EP-A1- 2 394 695
- EP-A1- 2 457 612
- WO-A2-2012/073097
- US-A1- 2001 023 367
- US-B1- 7 340 288

## Description

L'invention concerne les sondes intracardiaques de stimulation du ventricule gauche.

L'invention se situe dans le contexte général des "dispositifs médicaux implantables actifs" tels que définis par la directive 90/385/CEE du 20 juin 1990 du Conseil des communautés européennes, notamment les implants permettant de surveiller en continu le rythme cardiaque et délivrer si nécessaire au coeur des impulsions électriques de stimulation, de resynchronisation ou de défibrillation.

On fera principalement référence dans la présente description à des sondes intracardiaques "de stimulation", c'est-à-dire destinées à la délivrance d'impulsions à basse énergie utilisées pour les thérapies antibradycardiques ou de resynchronisation. Mais l'invention s'applique également aux sondes intracardiaques de cardioversion/défibrillation destinées à délivrer au coeur des chocs électriques de haute énergie pour tenter de mettre fin à une tachyarythmie. Sauf indication contraire, les termes génériques de "sonde (ou électrode) de stimulation", ou "de stimulation/défibrillation" viseront tout type de sonde utilisable à ces fins.

Pour la stimulation du ventricule droit, il suffit d'implanter une sonde endocavitaire par le réseau veineux périphérique droit. En revanche, pour stimuler le ventricule gauche, la situation est plus complexe.

La solution la plus souvent retenue consiste à introduire une sonde non pas dans la cavité à stimuler mais dans le réseau coronarien, la sonde étant pourvue d'une électrode qui sera appliquée contre la paroi de l'épicarde et orientée vers le ventricule gauche. Ces sondes stimulent le muscle cardiaque via une ou plusieurs électrodes ponctuelles dont la position est fonction de la trajectoire prédéfinie de la veine canulée.

Une sonde de ce type est par exemple le modèle *Situs LV,* commercialisé par Sorin CRM (Clamart, France) et décrit dans le EP 0 993 840 A1 (ELA Medical).

L'introduction d'une telle sonde se fait par le sinus coronaire, depuis son débouché dans l'oreillette droite. La sonde est ensuite poussée et orientée le long du réseau des veines coronaires jusqu'au site choisi. Une fois la veine cible atteinte, le chirurgien recherche un site de stimulation satisfaisant, avec un bon contact électrique de l'électrode de stimulation contre le tissu de l'épicarde, ce contact devant être maintenu malgré les variations ou sollicitations diverses au cours du temps.

Cette technique d'implantation n'est cependant pas toujours réalisable, notamment lorsque la conformation du sinus coronaire est trop accidentée, ou en cas de thrombose. En effet, le positionnement précis de l'électrode ou des électrodes destinée(s) à stimuler le ventricule gauche au travers de la paroi du myocarde est un paramètre critique, et il n'est pas toujours possible d'atteindre les sites de stimulation efficaces.

Une autre technique, plus difficile à mettre en oeuvre et présentant un caractère beaucoup plus invasif, consiste à implanter des électrodes épicardiques sur la paroi externe du myocarde, en un ou plusieurs sites appropriés disposés face à la cavité du ventricule gauche. Une variante de cette technique, exposée dans le EP 2 308 550 A1 (Sorin CRM), consiste à implanter l'électrode, constituée par la vis hélicoïdale conductrice d'une sonde à vis, via un cathéter coudé inséré dans le sac péricardique. Ces techniques sont toutefois relativement invasives et en outre généralement irréversibles, dans la mesure où il est très difficile de modifier le site d'implantation initialement choisi, et d'explanter si besoin la sonde dans une phase ultérieure.

Une autre approche, à laquelle se rattache l'invention, consiste à stimuler le ventricule gauche en appliquant des impulsions de stimulation à la paroi du septum interventriculaire (c'est-à-dire à la cloison séparant le ventricule gauche et le ventricule droit), au moyen d'une sonde introduite dans le ventricule droit selon une approche conventionnelle.

Cette technique implique de percer le septum interauriculaire ou interventriculaire, puis d'introduire une sonde traversant ce septum jusqu'à venir en contact avec un point de la paroi du ventricule gauche : les impulsions de stimulation seront alors appliquées directement au site endocavitaire gauche ainsi choisi.

Pour un exemple de technique de ponction transseptale contrôlée, on pourra se référer au EP 1 516 644 A1 (ELA Medical), qui décrit un accessoire de guidage comprenant une extrémité terminée par une vis d'ancrage à la paroi droite du septum, et dans lequel est introduit un mandrin perceur destiné à amorcer une ponction du septum. Après retrait de ce mandrin perceur, un mandrin de guidage est introduit dans l'accessoire, pour former un guide axial qui permettra ensuite d'introduite la sonde de stimulation, pourvue de son électrode, dans la cavité gauche après retrait de l'accessoire.

Une autre technique de réalisation d'une ponction transseptale, décrite par le EP 2 327 366 A1 (Sorin CRM), consiste à ancrer une vis à la paroi du septum, puis à appliquer à cette vis une énergie radiofréquence pour la faire progressivement s'enfoncer dans la paroi du septum jusqu'à traverser ce dernier. La ponction peut être également réalisée par un fil-guide alimenté par l'énergie RF, qui est poussé jusqu'à traverser de part en part le septum.

Ces techniques présentent divers inconvénients, essentiellement dus au fait qu'elles imposent de réaliser dans la paroi septale une ponction de diamètre suffisante pour pouvoir y introduire un cathéter-guide destiné à établir une communication entre cavités droite et gauche au travers de la paroi, pour pouvoir y introduire ensuite la sonde de stimulation endocavitaire gauche. Du fait que l'élément débouchant dans le ventricule gauche est un cathéter creux, il en résulte des risques importants d'embolie gazeuse. Pour éviter ce risque, il est impératif de prendre de nombreuses précautions lors du maniement des valves hémostatiques, de respecter des procédures de purge du matériel, etc. Toutefois, compte tenu du caractère très invasif de la procédure, des incertitudes demeurent sur le comportement du dispositif à long terme dans la circulation artérielle, ce qui implique un traitement anticoagulant pour éviter tout risque thromboembolique postopératoire. Enfin, toute extraction ultérieure de la sonde est pratiquement exclue, du fait des risques trop importants qui seraient encourus au niveau de la traversée du septum.

En tout état de cause, ces techniques sont très délicates à mettre en oeuvre et requièrent une grande habileté du praticien qui, avant de traverser le septum, doit toujours s'assurer du positionnement parfait de l'aiguille de perçage sur la paroi, la traversée du septum ne devant être entreprise que s'il ne subsiste aucun doute sur la position de l'aiguille, afin d'éviter tout risque de dissection accidentelle des parois par un mouvement inopiné de l'aiguille de perçage du septum - d'où le développement de nécessaires de perçage spécifiques, comme ceux décrits par les EP 1 516 644 A1 et EP 2 327 366 A1 précités.

Une autre technique encore, destinée à réduire ces risques, est exposée par les EP 2 457 612 A1 et EP 2 384 784 (Sorin CRM). L'idée de base consiste à supprimer le cathéter-guide associé à une sonde traversant le septum, et à remplacer cet ensemble par une sonde de type conventionnel vissée sur la paroi du ventricule droit au niveau du septum, en prolongeant la sonde par un microcâble transseptal, partiellement isolé et poussé dans le ventricule gauche jusqu'à venir en contact contre une cible située dans ce ventricule, par exemple contre la paroi libre de ce dernier (c'est-à-dire la paroi située à l'opposé de la cloison septale).

Du fait de l'extrême finesse de la ponction (qui a la dimension du diamètre du microcâble), cette technique réduit grandement les risques liés aux techniques antérieures. En revanche, dans la mesure où le microcâble est déployé librement dans le ventricule gauche, il est difficile de maitriser la délivrance des impulsions de stimulation en des sites précis, ainsi que de garantir un contact satisfaisant et permanent entre les électrodes de cette partie libre et la paroi du ventricule gauche, du fait de l'instabilité physique du microcâble sur la paroi libre résultant de la très grande flexibilité de ce microcâble. En outre, la transmission de la poussée de ponction via le microcâble peut être difficile : en effet, le besoin d'endurance mécanique impose une très grande souplesse pour le microcâble, souplesse qui est peu compatible avec l'exigence de "pushabilité" (aptitude à transmettre des efforts de poussée axiale appliqués depuis l'extrémité proximale) nécessaire lors de l'étape de ponction.

L'idée de base de l'invention, pour remédier à ces divers inconvénients et limitations, consiste à prolonger une sonde à vis, ancrée sur la paroi droite du septum interventriculaire, par une aiguille télescopique très fine, donc très peu invasive. L'aiguille est déployée de manière à ponctionner l'épaisseur du septum, mais sans émerger, ou très peu, du côté de la paroi gauche. La vis est en principe passive (elle n'a qu'un rôle mécanique) mais l'extrémité de l'aiguille est active, avec une ou plusieurs électrodes capables d'atteindre précisément la branche gauche du faisceau de His : cette branche constitue en effet une ligne de conduction rapide, permettant de stimuler efficacement et sans délai le ventricule gauche même en cas, par exemple, de bloc gauche local. Comme on le verra, la sonde est pourvue de moyens de réglage précis de la profondeur de ponction, permettant d'atteindre la zone cible recherchée de façon parfaitement progressive et maitrisée, au surplus en ne faisant intervenir que des gestes opératoires conventionnels, habituels pour le praticien.

Une technique telle que celle enseignée par les EP 2 457 612 A1 et EP 2 384 784 précités, qui est destinée à l'implantation d'un microcâble poussé, au travers du septum, dans le ventricule gauche jusqu'à venir en contact contre une cible située dans ce ventricule (par exemple contre la paroi libre de ce dernier, située à l'opposé de la cloison septale) n'est cependant pas transposable à l'implantation, comme dans le cas présent, d'une aiguille de stimulation intraseptale et non plus transseptale.

En effet, dans la mesure où la stimulation doit intervenir au niveau du septum, il est nécessaire d'ajuster très précisément la position de l'extrémité de l'aiguille, c'est-à-dire de sa partie active portant la(les) électrode(s) de stimulation, qui doi(ven)t se situer dans l'épaisseur du septum après implantation. Ceci n'est pas possible avec une technique consistant à simplement pousser un microcâble dans une gaine, technique qui est insuffisante pour garantir la précision millimétrique requise pour une implantation intraseptale.

Les WO 2012/073097 A2 et US 2001/0023367 A1 décrivent des mécanismes d'insertion d'aiguilles dans des contextes différents, non liés à la stimulation cardiaque.

Pour atteindre les buts exposés ci-dessus, l'invention propose une sonde du type général divulgué par le EP 2 457 612 A1 précité, c'est-à-dire comportant : un corps de sonde avec une gaine creuse souple isolée, comportant une lumière centrale logeant un conducteur interne mobile axialement et en rotation dans la lumière ; côté proximal, un connecteur électrique de couplage à un générateur d'un dispositif médical implantable actif, ce connecteur comportant au moins une broche centrale reliée au conducteur interne ; et, côté distal, une tête de sonde avec un corps tubulaire solidaire de la gaine et prolongé côté distal par une vis hélicoïdale d'ancrage saillante et solidaire du corps de sonde.

La tête de sonde est apte à s'étendre dans la cavité du ventricule droit et la vis d'ancrage étant apte à pénétrer dans la paroi droite du septum interventriculaire sous l'effet d'un mouvement de vissage imprimé à la gaine du corps de sonde depuis l'extrémité proximale de la sonde.

La sonde comprend en outre une aiguille de stimulation électriquement reliée au conducteur interne et comprenant à son extrémité distale une partie libre active avec au moins une électrode de stimulation apte à appliquer des impulsions de stimulation. Cette aiguille est une aiguille télescopique mobile axialement entre une position rétractée à l'intérieur du corps tubulaire, et une position totalement ou partiellement déployée avec la partie libre active de l'aiguille émergeant hors du corps tubulaire.

De façon caractéristique de l'invention, afin de permettre une stimulation intraseptale par l'aiguille, où lesdites impulsions sont appliquées au septum dans une région voisine de la paroi gauche de celui-ci, la sonde comprend en outre un mécanisme de manoeuvre permettant de produire un déplacement contrôlé de l'aiguille, de sa position rétractée à sa position partiellement ou totalement déployée, sous l'effet d'un mouvement de rotation relative de la broche du connecteur par rapport au corps de sonde, mouvement imprimé à cette broche depuis l'extrémité proximale de la sonde. Le mécanisme de manoeuvre comprend un noyau de guidage et d'entrainement solidaire de l'aiguille en rotation et en translation, et mobile en translation et en rotation à l'intérieur du corps tubulaire, et des moyens de couplage du noyau au corps tubulaire, aptes à transformer un mouvement de rotation relative du noyau par rapport au corps tubulaire en un mouvement de translation axiale du noyau à l'intérieur du corps tubulaire. Selon diverses caractéristiques subsidiaires avantageuses :
- les moyens de couplage sont des moyens de type vis-écrou comprenant un filet hélicoïdal interne du corps tubulaire coopérant avec un organe conjugué de venue en prise du noyau avec le filet du corps tubulaire, notamment un filet hélicoïdal porté par une surface externe du noyau ;
- le conducteur interne est un conducteur bobiné disposé dans une région périphérique de la gaine creuse ;
- l'amplitude du mouvement de translation axiale du noyau à l'intérieur du corps tubulaire est comprise entre 10 et 15 mm ;
- la longueur de la partie émergente de l'aiguille en position déployée est comprise entre 0 et 15 mm, et son diamètre est d'au plus 1,5 French (0,5 mm) ;
- la surface extérieure de l'aiguille est électriquement isolée, à l'exception d'au moins une zone dénudée, située dans la partie libre active, formant ladite au moins une électrode de stimulation ;
- la partie libre active comprend la au moins une zone dénudée s'étend sur au plus 6 mm à partir de l'extrémité distale libre de l'aiguille ;
- la sonde comprend une pluralité de zones dénudées distinctes s'étendant en succession le long de la partie libre active et séparées par des zones intercalaires isolantes non dénudées ;
- la surface totale de la(des) zone(s) dénudée(s) de la partie libre active est d'au plus 6 mm² ;
- à sa partie proximale, l'aiguille est prolongée par une tige dont elle est solidaire et qui est soudée au conducteur interne ;
- la vis d'ancrage et/ou le corps tubulaire sont électriquement inactifs.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 est une vue en coupe schématique du myocarde, montrant ses différentes cavités ainsi que les principales voies de conduction électrique.

Les Figures 2 et 3 sont des vues en coupe de la tête de sonde, respectivement dans la position rétractée et dans la position déployée de l'aiguille de stimulation.

Les Figures 4a à 4e illustrent les différentes étapes de l'implantation dans la paroi du septum interventriculaire de la sonde selon l'invention.

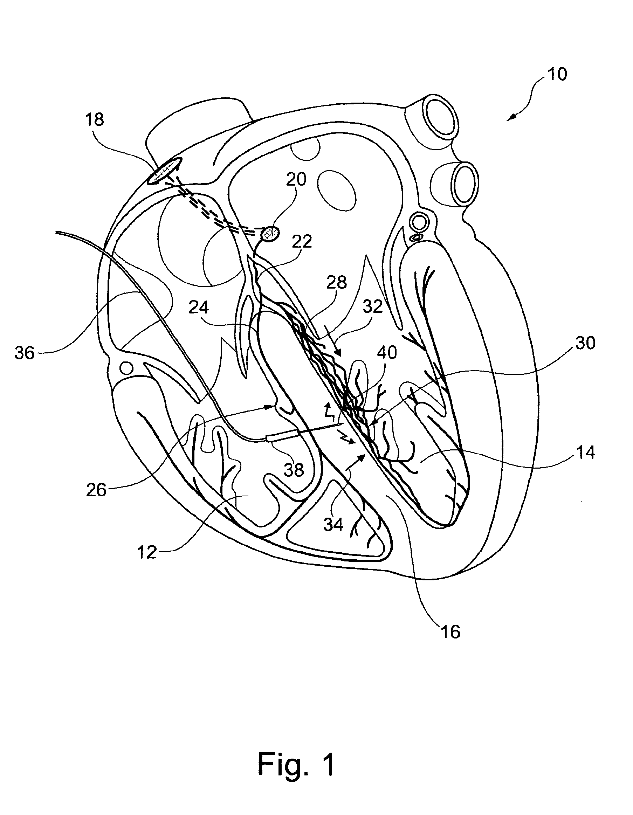

La Figure 1 représente schématiquement, en coupe, le myocarde 10 avec le ventricule droit 12 et le ventricule gauche 14 séparés par le septum interventriculaire 16. Celui-ci présente une épaisseur typique de l'ordre de 10 à 15 mm et constitue une partie significative de la masse cardiaque.

Les ondes de dépolarisation qui prennent naissance dans le noeud sinoatrial 18 sont transmises au noeud atrioventriculaire 20 puis au faisceau de His 22 qui se divise en deux branches s'étendant le long du septum 16, avec une branche droite 24 dans la région de la paroi septale droite 26 et une branche gauche 28 dans la région de la paroi septale gauche 30.

En particulier, la branche gauche 28 constitue une ligne de conduction électrique longitudinale rapide (flèche 32), avec une vitesse de l'ordre de 4 m/s. Or les techniques conventionnelles mettant en oeuvre des sondes à vis rétractable permettent de stimuler la paroi droite 26, de sorte que pour stimuler le ventricule gauche 14 les ondes de dépolarisation doivent traverser le septum 16 (flèche 34), mais avec une vitesse de conduction transversale relativement faible, de l'ordre de 0,4 m/s : ceci introduit un retard d'environ 30 ms entre l'application de l'impulsion sur la paroi droite 26 et l'excitation de la branche gauche 28 conduisant à la contraction du ventricule gauche 14.

On comprend ainsi qu'un déplacement du point de stimulation de la paroi droite du septum (technique conventionnelle) vers la paroi gauche du septum, à proximité immédiate de la branche gauche 28 du faisceau de His (technique de l'invention) peut contribuer sensiblement à la réduction du délai entre l'application de l'impulsion et la contraction effective du ventricule gauche, et/ou pallier les effets d'un bloc gauche local.

Selon l'invention, on utilise à cet effet une sonde 36, implantée de manière conventionnelle dans les cavités droites, avec une tête de sonde 38 ancrée contre la paroi droite 26 du septum 16. Cette tête de sonde est prolongée par une aiguille télescopique dont l'extrémité distale 40 porte les électrodes de stimulation. Une ponction progressive et maitrisée du septum permet de placer l'extrémité 40 de l'aiguille au voisinage de la paroi gauche 30 sans traverser le septum, ou en ne débouchant qu'à peine de cette paroi, de manière à stimuler directement la branche gauche 28 du faisceau de His précisément dans la zone de conduction rapide, assurant ainsi une stimulation directe et sans délai du ventricule gauche.

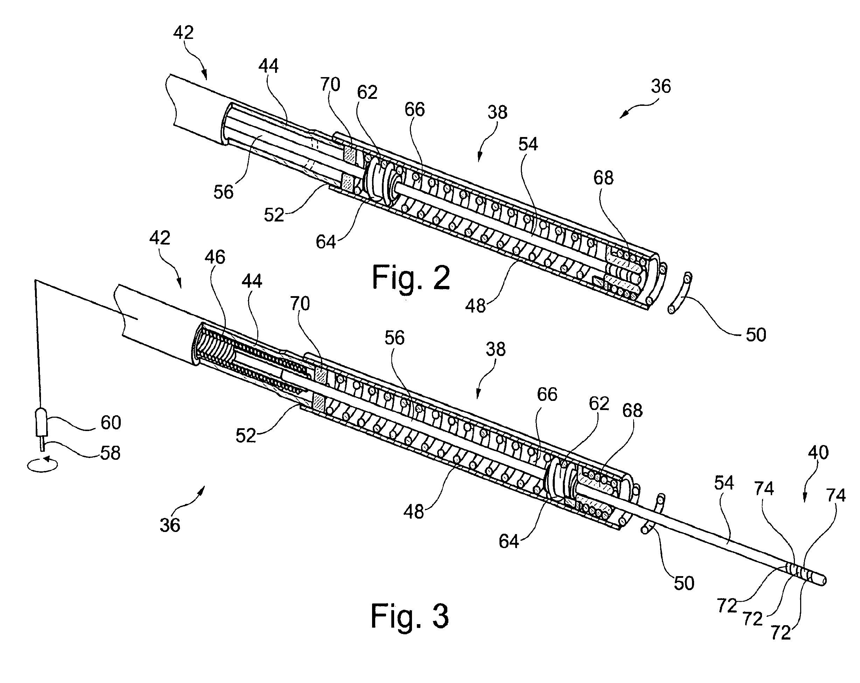

Les Figures 2 et 3 illustrent plus précisément, respectivement dans la position rétractée et dans la position déployée de l'aiguille télescopique, la structure la tête de sonde 38 située à l'extrémité distale de la sonde 36, et destinée à venir en appui contre la paroi septale droite.

Le corps de sonde 42 comprend une gaine creuse souple isolée 44, par exemple en un matériau tel que le polyuréthanne qui présente de très bonnes propriétés de glisse lorsque la gaine est introduite dans un cathéter de mise en place, et de "torquabilité", c'est-à-dire d'aptitude à transmettre jusqu'à l'extrémité distale un couple de torsion imprimé depuis l'extrémité proximale de la sonde. La lumière interne de la gaine creuse 44 loge un ou plusieurs conducteurs internes, par exemple comme dans l'exemple illustré un conducteur bobiné 46 disposé dans une région périphérique de la gaine creuse de manière à laisser subsister un espace central libre, notamment pour l'insertion d'un mandrin de guidage et/ou de rigidification à l'intérieur de la gaine. Si la gaine comporte plusieurs conducteurs internes, la configuration peut être avantageusement du type "coradiale", où les deux (ou plus) conducteurs étant des conducteurs isolés bobinés côte-à-côte dans une région périphérique de la lumière interne de la gaine creuse 44, et formant un bobinage de rayon unique sur une seule épaisseur.

La gaine 44 de la sonde est prolongée à son extrémité distale par un corps tubulaire 48, présentant un diamètre extérieur de l'ordre de 7 French (2,33 mm). Ce corps tubulaire 48 porte à son extrémité distale une vis hélicoïdale d'ancrage 50, saillante sur une longueur axiale de l'ordre de 2 mm. Cette vis hélicoïdale est solidaire du corps tubulaire 48 qui, à son extrémité proximale opposée est solidarisé en 52 à la gaine creuse 44. De la sorte, tout mouvement de rotation du corps de sonde sous l'effet notamment d'un couple appliqué depuis l'extrémité proximale de la sonde par le praticien, sera intégralement transmis à la vis hélicoïdale 50 via le corps tubulaire 48, ces trois éléments étant solidaires entre eux.

Une sonde à vis implantée semblable à ce que l'on vient de décrire est généralement utilisée comme sonde de détection/stimulation après ancrage de la vis au site de stimulation endocavitaire.

Dans le cas de l'invention tel n'est pas le cas : la fonction de la vis est ici de servir de moyen de support et de guidage d'une aiguille de stimulation ponctionnée dans la paroi du septum. Pour la mise en oeuvre de l'invention, la vis 50 et le corps tubulaire 48 ne sont pas (ou pas nécessairement) des éléments électriquement actifs.

Le corps tubulaire 48 loge, en position rétractée (Figure 2), une aiguille télescopique de stimulation 54 mobile axialement en translation par rapport au corps tubulaire 48, entre la position rétractée de la Figure 2 et une position déployée illustrée Figure 3. Côté proximal, l'aiguille télescopique 54 est prolongée par une tige de manoeuvre axiale 56 soudée côté proximal à l'extrémité distale du conducteur interne 46, de sorte que tout déplacement axial ou en rotation de ce conducteur interne 46 est intégralement transmis à l'aiguille 54. De plus, l'aiguille 54 est non seulement mécaniquement, mais aussi électriquement reliée au conducteur interne 46.

Plus précisément, l'extrémité proximale du conducteur interne 46 est reliée à une broche 58 d'un connecteur 60 de couplage à un boitier de générateur d'impulsions. Il s'agit par exemple d'un connecteur standard de type IS-1 ou autre, avec une broche 58 mobile en rotation par rapport au corps du connecteur 60, de manière à permettre une manipulation de type *pin-driven,* où le praticien maintient d'une main le corps du connecteur 60 (solidaire de la gaine 44 du corps de sonde 42) et fait tourner de l'autre main, directement ou par l'intermédiaire d'un outil, la broche (*pin*) 58 de ce connecteur. La broche 58 étant solidaire du conducteur axial 46, lui-même libre en rotation à l'intérieur de la gaine creuse 44, le mouvement de la broche est directement transmis à la tige de manoeuvre 56 et à l'aiguille télescopique 54.

L'aiguille télescopique 54 est une aiguille pleine, de manière à ne présenter aucun élément creux susceptible de déboucher dans le ventricule gauche au cas où l'aiguille traverse le septum de part en part, donc ne créant aucun risque d'embolie gazeuse.

Le guidage et le déploiement de l'aiguille télescopique 54 sont contrôlés par un élément 62 mobile à l'intérieur du corps tubulaire 48 et formant noyau de guidage et d'entrainement. Ce noyau 62, solidaire de la tige de manoeuvre 56, est couplé au corps tubulaire par exemple par un filet externe 64 solidaire du noyau 62, coopérant avec un filet interne 66 solidaire du corps tubulaire 44. De la sorte, un mouvement de rotation axiale relative de la tige de manoeuvre 56 par rapport au corps tubulaire 48 (résultant d'un mouvement correspondant de rotation relative de la broche 58 par rapport au connecteur 60 (côté proximal) se traduira par une translation axiale du noyau d'entrainement 62, et donc de l'aiguille télescopique 54 par rapport au corps tubulaire 48. L'amplitude de cette translation axiale est typiquement de l'ordre de 10 à 15 mm, soit une valeur notablement supérieure à celle d'un mécanisme conventionnel de déploiement de la vis d'une sonde à vis rétractable.

Pour assurer le guidage de l'aiguille et l'étanchéité du volume intérieur du corps tubulaire et de la lumière de la gaine creuse 44, des paliers souples 68 et 70 sont prévus respectivement côté distal et côté proximal du corps tubulaire 48.

À l'état rétracté (Figure 2), l'aiguille 54 est logée entièrement à l'intérieur du corps tubulaire 48, dont ne dépasse que la vis hélicoïdale d'ancrage 50, qui est fixe par rapport au corps tubulaire 48.

À l'état totalement déployé (Figure 3), l'aiguille télescopique 54 émerge du corps tubulaire sur une longueur d'environ 15 mm.

L'aiguille peut être déployée de manière contrôlée entre n'importe quelle de ces positions extrêmes, donc sur une longueur comprise entre 0 et 15 mm, de façon ajustable par le praticien.

L'aiguille télescopique 54 est réalisée en un matériau conducteur de diamètre typique 1 French (0,33 mm). Le matériau peut être notamment un alliage inoxydable tel que le MP35N, ou être constitué d'une structure composite, par exemple avec une âme en MP35N et un revêtement périphérique biocompatible et radio-opaque tel que le platine ou un alliage de platine.

En variante, l'aiguille de stimulation intraseptale 54 peut être réalisée à partir d'un microcâble présentant un diamètre de l'ordre de 1,5 F (0,5 mm), ce qui permet de bénéficier de la relative souplesse de celui-ci par rapport à une configuration monofilament de même diamètre, avec de ce fait une meilleure tenue en fatigue. Un tel microcâble peut comprendre un coeur constitué d'une pluralité de brins composites toronnés ensemble, avec par exemple un brin central entouré de six brins périphériques. Chaque brin composite est lui-même constitué d'un fil d'âme central en platine iridié (pour la radio-opacité) entouré d'une pluralité de fils également composites procurant les propriétés mécaniques et électriques recherchées, avec par exemple un coeur d'argent (pour la conductivité électrique) enveloppé de nitinol (pour les propriétés de résistance aux contraintes mécaniques). Ces différents fils sont disponibles dans le commerce, par exemple auprès de la Société Fort Wayne Metals Inc., Fort Wayne, USA, et sont utilisés dans le domaine médical notamment pour réaliser des conducteurs de défibrillation.

L'aiguille télescopique de stimulation 54 est revêtue d'un matériau électriquement isolant, par exemple le parylène. Pour constituer la partie libre active 40, le revêtement isolant de l'aiguille est localement retiré de manière à faire apparaitre une ou plusieurs régions dénudées 72 séparées par des régions isolantes 74. Avantageusement, la partie libre active 40 comportant ces régions dénudées 72 s'étend sur une longueur de l'ordre de 6 mm, la surface des régions dénudées étant d'au plus 6 mm² de manière à limiter la surface stimulante.

Le fait de disposer de plusieurs électrodes 72 sur une longueur relativement importante (6 mm) offre la possibilité de compenser les variations d'épaisseur du septum qui se contracte au cours d'un cycle cardiaque, et donc de maintenir une surface stimulante en vis-à-vis des tissus.

Du point de vue électrique, la vis d'ancrage 50 et le corps tubulaire 48 sont en principe inactifs, et revêtus d'un isolant tel que le parylène sur la totalité de leur surface. Dans certaines configurations particulières, il est toutefois possible de rendre électriquement actifs la vis d'ancrage 50 et/ou tout ou partie du corps tubulaire 48, par exemple pour permettre une stimulation simultanée au niveau de la vis d'ancrage (paroi droite du septum) et de la partie libre active 40 de l'aiguille télescopique (paroi gauche du septum).

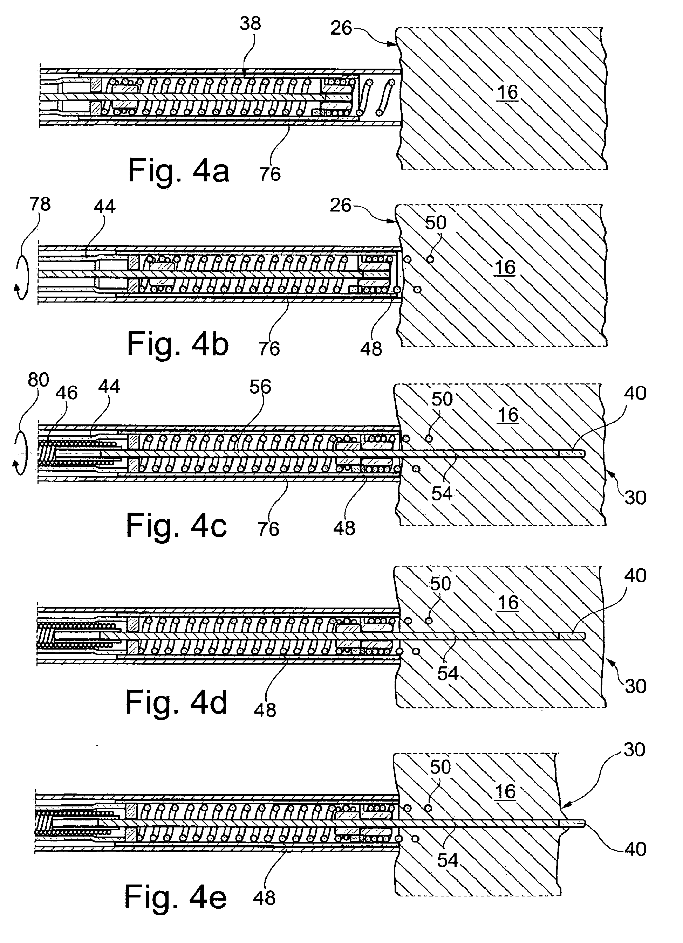

On va maintenant décrire, en référence aux Figures 4a à 4e, la manière d'implanter la sonde que l'on vient de décrire.

Au préalable, la sonde a été introduite dans un cathéter 76 de type classique, destiné au prépositionnement de la tête de sonde contre la paroi du septum ventriculaire. Ce cathéter présente un diamètre extérieur typique de 9 French (3 mm). L'utilisation d'un cathéter permet de protéger la vis d'ancrage 50 (qui n'est pas une vis télescopique) lors du passage dans les vaisseaux et du franchissement de la valve tricuspide. Des exemples de cathéters appropriés permettant un accès à la paroi droite du septum interventriculaire sont décrits par exemple dans les EP 2 135 638 A1 (cathéter préformé auto-orientable dans la direction de la paroi du septum) et FR 2 932 688 A1 (cathéter bimatière dont l'extrémité est conformable à volonté), tous deux au nom de ELA Medical.

La première phase consiste à repérer le site d'ancrage, en manipulant l'ensemble sonde-cathéter pour l'introduire dans la veine cave supérieure, l'oreillette droite puis le ventricule droit 12 jusqu'à venir en butée contre la paroi droite 26 du septum interventriculaire 16.

La Figure 4a illustre la configuration atteinte lorsque le cathéter 76 est mis en appui contre la paroi droite du septum 16, avec la tête de sonde 38 glissée à l'intérieur du cathéter 76 jusqu'au voisinage de cette paroi 26.

L'étape suivante, illustrée Figure 4b, consiste à imprimer à la gaine 44 du corps de sonde un mouvement de rotation (flèche 78) depuis l'extrémité proximale de ce corps de sonde. Cette rotation a pour effet de faire pénétrer la vis 50 dans les tissus du septum 16 sur une relativement faible profondeur (de l'ordre de 2 mm) et d'ancrer ainsi le corps tubulaire 48, dont cette vis est solidaire, contre la paroi droite 26 du septum. Le vissage complet est détecté tactilement par le praticien par la résistance opposée à la rotation.

Le site atteint est confirmé par examen radiographique selon différentes incidences ; si la position n'est pas satisfaisante, le praticien peut dévisser la tête de sonde et déplacer celle-ci sous contrôle vers un autre point, puis tester le nouveau site.

Une fois le corps tubulaire 48 ainsi amarré à la paroi du septum, le praticien imprime une rotation à la broche 58 du connecteur 60. Cette rotation, transmise par le conducteur interne 46 (flèche 80, Figure 4c), est appliquée à la tige de manoeuvre 56 ce qui, par voie de conséquence, provoque la pénétration progressive de l'aiguille de stimulation télescopique 54 dans l'épaisseur du septum 16, dans la mesure où le corps de sonde, et donc la gaine 44 et le corps tubulaire 48, sont maintenus immobiles pendant ce déploiement.

Cette manoeuvre est exécutée sous amplificateur de brillance, de manière à visualiser la position de la partie active 40 par rapport aux parois du septum (qui peuvent être éventuellement visualisées par injection de produit de contraste via le cathéter 76). Le praticien peut également procéder à ce stade à un *mapping* ou test électrique pour vérifier l'efficacité du site choisi, de manière à déterminer le positionnement optimum de la partie active 40 de l'aiguille, c'est-à-dire le degré de déploiement de celle-ci.

Si le site initialement choisi n'est pas satisfaisant, il est possible d'opérer un ou plusieurs repositionnements. En effet, l'ancrage de la vis 50 sur une relativement faible profondeur (de l'ordre de 2 mm, pour une épaisseur typique de septum interventriculaire de 10 à 15 mm) et le diamètre extrêmement réduit de la ponction (du fait du très faible diamètre de l'aiguille 55, de l'ordre de 0,3 mm) permettent de retirer la sonde sans créer de dommages irréversibles au septum - et ceci à l'inverse d'une approche transseptale classique, moins tolérante à l'erreur du fait de la taille de la ponction subsistante (correspondant à un diamètre de cathéter, typiquement de 9 French soit 2,3 mm).

Le degré plus ou moins important de déploiement de l'aiguille télescopique 54 permet de prendre en compte non seulement les variations d'épaisseur du septum selon la région d'implantation, mais aussi de tenir compte du fait que la sonde peut se trouver positionnée dans une position plus ou moins perpendiculaire à la paroi du septum (qui, en coupe transversale, présente une forme bombée).

Une fois sélectionnés le site définitif et le degré de déploiement de l'aiguille télescopique, le praticien retire le cathéter 76, aboutissant ainsi à la configuration définitive de la tête de sonde.

Dans la configuration illustrée sur la Figure 4d, l'extrémité distale de l'aiguille 54 (avec la partie active 40) n'émerge pas de la paroi gauche 30 du septum.

Toutefois, comme illustré Figure 4e, il peut être intéressant de traverser complètement le septum afin de positionner l'extrémité libre 40 de l'aiguille 54 pour que celle-ci émerge très légèrement dans la cavité du ventricule gauche. Ceci permet d'éviter un effet de pilonnage de l'extrémité de l'aiguille sur les tissus cibles, pouvant conduire à leur destruction et donc à une perte de capture lors de la stimulation.

## Revendications

1. Une sonde intracardiaque de stimulation du ventricule gauche, comprenant :
- un corps de sonde (42) avec une gaine creuse souple isolée (44), comportant une lumière centrale logeant un conducteur interne (46) mobile axialement et en rotation dans la lumière ;
- côté proximal, un connecteur électrique (60) de couplage à un générateur d'un dispositif médical implantable actif, ce connecteur comportant au moins une broche centrale (58) reliée au conducteur interne ;
- côté distal, une tête de sonde avec un corps tubulaire (48) solidaire de la gaine et prolongé côté distal par une vis hélicoïdale d'ancrage (50) saillante et solidaire du corps de sonde,
la tête de sonde étant apte à s'étendre dans la cavité du ventricule droit (12) et la vis d'ancrage étant apte à pénétrer dans la paroi droite (26) du septum interventriculaire (16) sous l'effet d'un mouvement de vissage (78) imprimé à la gaine du corps de sonde depuis l'extrémité proximale de la sonde ;
- une aiguille de stimulation (54) électriquement reliée au conducteur interne et comprenant à son extrémité distale une partie libre active (40) avec au moins une électrode de stimulation (72) apte à appliquer des impulsions de stimulation,
cette aiguille étant une aiguille télescopique mobile axialement entre une position rétractée à l'intérieur du corps tubulaire, et une position totalement ou partiellement déployée avec la partie libre active de l'aiguille émergeant hors du corps tubulaire,
sonde ***caractérisée en ce que**,* afin de permettre une stimulation intraseptale par l'aiguille, où lesdites impulsions sont appliquées au septum (16) dans une région voisine de la paroi gauche (30) de celui-ci,
la sonde comprend en outre :
- un mécanisme de manoeuvre (62, 64, 66) apte à produire un déplacement contrôlé de l'aiguille, de sa position rétractée à sa position partiellement ou totalement déployée, sous l'effet d'un mouvement de rotation relative de la broche du connecteur par rapport au corps de sonde, mouvement imprimé à cette broche depuis l'extrémité proximale de la sonde,
ce mécanisme de manoeuvre comprenant :
- un noyau (62) de guidage et d'entrainement solidaire de l'aiguille en rotation et en translation, et mobile en translation et en rotation à l'intérieur du corps tubulaire ; et
- des moyens de couplage (64, 66) du noyau au corps tubulaire, aptes à transformer un mouvement de rotation relative du noyau par rapport au corps tubulaire en un mouvement de translation axiale du noyau à l'intérieur du corps tubulaire.

2. La sonde de la revendication 1, dans laquelle lesdits moyens de couplage sont des moyens de type vis-écrou comprenant un filet hélicoïdal interne (66) du corps tubulaire coopérant avec un organe conjugué (64) de venue en prise du noyau avec le filet du corps tubulaire.

3. La sonde de la revendication 2, dans laquelle l'organe conjugué de venue en prise du noyau est un filet hélicoïdal (64) porté par une surface externe du noyau.

4. La sonde de la revendication 1, dans laquelle le conducteur interne (46) est un conducteur bobiné disposé dans une région périphérique de la gaine creuse.

5. La sonde de la revendication 1, dans laquelle l'amplitude du mouvement de translation axiale du noyau à l'intérieur du corps tubulaire est comprise entre 10 et 15 mm.

6. La sonde de la revendication 1, dans laquelle la longueur de la partie émergente de l'aiguille en position déployée est comprise entre 0 et 15 mm.

7. La sonde de la revendication 1, dans laquelle le diamètre de l'aiguille est d'au plus 1,5 French (0,5 mm).

8. La sonde de la revendication 1, dans laquelle la surface extérieure de l'aiguille est électriquement isolée, à l'exception d'au moins une zone dénudée (72), située dans la partie libre active (40), formant ladite au moins une électrode de stimulation.

9. La sonde de la revendication 8, dans laquelle la partie libre active (40) comprenant ladite au moins une zone dénudée s'étend sur au plus 6 mm à partir de l'extrémité distale libre de l'aiguille.

10. La sonde de la revendication 8, comprenant une pluralité de zones dénudées (72) distinctes s'étendant en succession le long de la partie libre active et séparées par des zones intercalaires isolantes (74) non dénudées.

11. La sonde de la revendication 8, dans laquelle la surface totale de la(des) zone(s) dénudée(s) de la partie libre active est d'au plus 6 mm².

12. La sonde de la revendication 1, dans laquelle à sa partie proximale l'aiguille est prolongée par une tige (56) dont elle est solidaire et qui est soudée au conducteur interne (46).

13. La sonde de la revendication 1, dans laquelle la vis hélicoïdale d'ancrage (50) est électriquement inactive.

14. La sonde de la revendication 1, dans laquelle le corps tubulaire (48) est électriquement inactif.

## Patentansprüche

1. Intrakardiale Sonde zur Stimulation des linken Ventrikels, welche aufweist:
- einen Sondenkörper (42) mit einem isolierten flexiblen hohlen Mantel (44), der ein zentrales Lumen aufweist, das einen inneren Leiter (46) aufnimmt, der in dem Lumen axial beweglich und drehbeweglich ist;
- auf der proximalen Seite einen elektrischen Verbinder (60) zur Kopplung mit einem Generator eines aktiven implantierbaren medizinischen Geräts, wobei dieser Verbinder mindestens einen Mittelstift (58) aufweist, der mit dem inneren Leiter verbunden ist;
- auf der distalen Seite einen Sondenkopf mit einem rohrförmigen Körper (48), der mit dem Mantel fest verbunden ist und auf der distalen Seite durch eine vorstehende und mit dem Sondenkörper fest verbundene Verankerungs-Schneckenwendel (50) verlängert ist,
wobei der Sondenkopf geeignet ist, sich in den Hohlraum des rechten Ventrikels (12) hinein zu erstrecken, und die Verankerungswendel geeignet ist, unter der Wirkung einer Schraubbewegung (78), die vom proximalen Ende der Sonde aus auf den Mantel des Sondenkörpers ausgeübt wird, in die rechte Wand (26) des interventrikulären Septums (16) einzudringen;
- eine Stimulationsnadel (54), die mit dem inneren Leiter elektrisch verbunden ist und an ihrem distalen Ende einen aktiven freien Teil (40) mit mindestens einer Stimulationselektrode (72) aufweist, die geeignet ist, Stimulationsimpulse anzulegen,
wobei diese Nadel eine Teleskopnadel ist, die zwischen einer ins Innere des rohrförmigen Körpers zurückgezogenen Position und einer vollständig oder teilweise hinausbewegten Position, in welcher der aktive freie Teil der Nadel aus dem rohrförmigen Körper hervorragt, axial beweglich ist,
wobei die Sonde ***dadurch gekennzeichnet*** ist, dass, um eine intraseptale Stimulation durch die Nadel zu ermöglichen, bei der die Impulse an das Septum (16) in einem der linken Wand (30) desselben benachbarten Bereich angelegt werden,
die Sonde außerdem aufweist:
- einen Betätigungsmechanismus (62, 64, 66), der geeignet ist, eine kontrollierte Verschiebung der Nadel aus ihrer zurückgezogenen Position in ihre vollständig oder teilweise hinausbewegte Position unter der Einwirkung einer relativen Drehbewegung des Stifts des Verbinders in Bezug auf den Sondenkörper zu erzeugen, wobei die Bewegung diesem Stift vom proximalen Ende der Sonde aus verliehen wird,
wobei dieser Betätigungsmechanismus aufweist:
- einen Führungs- und Antriebskern (62), der mit der Nadel drehfest und verschiebefest verbunden ist und im Inneren des rohrförmigen Körpers translatorisch beweglich und drehbeweglich ist; und
- Mittel zur Kopplung (64, 66) des Kerns mit dem rohrförmigen Körper, die geeignet sind, eine relative Drehbewegung des Kerns bezüglich des rohrförmigen Körpers in eine axiale Translationsbewegung des Kerns im Inneren des rohrförmigen Körpers umzuwandeln.

2. Sonde nach Anspruch 1, wobei die Mittel zur Kopplung Mittel vom Typ Schraube-Mutter sind, die ein inneres Schraubengewinde (66) des rohrförmigen Körpers aufweisen, das mit einem komplementären Eingriffselement (64) zum Eingriff des Kerns mit dem Gewinde des rohrförmigen Körpers zusammenwirkt.

3. Sonde nach Anspruch 2, wobei das komplementäre Eingriffselement des Kerns ein Schraubengewinde (64) ist, das von einer Außenfläche des Kerns getragen wird.

4. Sonde nach Anspruch 1, wobei der innere Leiter (46) ein eine Spule bildender Leiter ist, der in einem Umfangsbereich des hohlen Mantels angeordnet ist.

5. Sonde nach Anspruch 1, wobei die Amplitude der axialen Translationsbewegung des Kerns im Inneren des rohrförmigen Körpers zwischen 10 und 15 mm liegt.

6. Sonde nach Anspruch 1, wobei die Länge des herausragenden Teils der Nadel in der hinausbewegten Position zwischen 0 und 15 mm liegt.

7. Sonde nach Anspruch 1, wobei der Durchmesser der Nadel höchstens 1,5 French (0,5 mm) beträgt.

8. Sonde nach Anspruch 1, wobei die Außenfläche der Nadel elektrisch isoliert ist, mit Ausnahme mindestens eines abisolierten Bereichs (72), der sich in dem aktiven freien Teil (40) befindet und die mindestens eine Stimulationselektrode bildet.

9. Sonde nach Anspruch 8, wobei sich der aktive freie Teil (40), der den mindestens einen abisolierten Bereich aufweist, von dem freien distalen Ende der Nadel aus auf höchstens 6 mm erstreckt.

10. Sonde nach Anspruch 8, welche mehrere verschiedene abisolierte Bereiche (72) aufweist, die sich aufeinander folgend entlang des aktiven freien Teils erstrecken und durch nicht abisolierte isolierende Zwischenbereiche (74) getrennt sind.

11. Sonde nach Anspruch 8, wobei die Gesamtfläche des (der) abisolierten Bereichs (Bereiche) des aktiven freien Teils höchstens 6 mm² beträgt.

12. Sonde nach Anspruch 1, wobei an ihrem proximalen Teil die Nadel durch einen Schaft (56) verlängert ist, mit dem sie fest verbunden ist und der an den inneren Leiter (46) angeschweißt ist.

13. Sonde nach Anspruch 1, wobei die Verankerungs-Schneckenwendel (50) elektrisch inaktiv ist.

14. Sonde nach Anspruch 1, wobei der rohrförmige Körper (48) elektrisch inaktiv ist.

## Claims

1. An endocardial lead for stimulation of the left ventricle, comprising:
- a lead body (42) with an insulated flexible hollow sheath (44), including a central lumen accommodating an inner conductor (46) axially and rotationally mobile within the lumen;
- on the proximal side, an electric connector (60) for the coupling to a generator of an active implantable medical device, this connector including at least one central pin (58) linked to the inner conductor;
- on the distal side, a lead head with a tubular body (48) fastened to the sheath and continued on the distal side by a protruding anchoring helical screw (50), fastened to the lead body,
the lead head being adapted to extend in the cavity of the right ventricle (12) and the anchoring screw being adapted to penetrate in the right wall (26) of the interventricular septum (16) under the effect of a screwing movement (78) imparted to the sheath of the lead body from the proximal end of the lead;
- a stimulation needle (54) electrically connected to the inner conductor and comprising at its distal end an active free part (40) with at least one stimulation electrode (72) adapted to apply stimulation pulses, this needle being a telescopic needle axially mobile between a retracted position, inside the tubular body, and a fully or partially extended position, with the active free part of the needle emerging out of the tubular body,
the lead being **characterized in that**, so as to allow an intraseptal stimulation by the needle, where said pulses are applied to the septum (16) in a neighbouring region of the left wall (30) of the latter,
the lead further comprises:
- an operating mechanism (62, 64, 66) adapted to produce a controlled displacement of the needle, from its retracted position to its partially or fully extended position, under the effect of a movement of relative rotation of the connector pin with respect to the lead body, such movement being imparted to this pin from the proximal end of the lead,
this operating mechanism comprising:
- a guiding and driving core (62) rotationally and translationally fastened to the needle, and translationally and rotationally mobile inside the tubular body; and
- means (64, 66) for coupling the core to the tubular body, adapted to transform a movement of relative rotation of the core with respect to the tubular body into a movement of axial translation of the core inside the tubular body.

2. The lead of claim 1, wherein said coupling means are means of the screw-nut type comprising an helical inner thread (66) of the tubular body, cooperating with a conjugated member (64) for engagement of the node with the thread of the tubular body.

3. The lead of claim 2, wherein the node-engagement conjugated member is an helical thread (64) carried by an outer surface of the core.

4. The lead of claim 1, wherein the inner conductor (46) is a winded conductor arranged in a peripheral region of the hollow sheath.

5. The lead of claim 1, wherein the amplitude of the movement of axial translation of the core inside the tubular body is comprised between 10 and 15 mm.

6. The lead of claim 1, wherein the length of the emerging part of the needle in the extended position is comprised between 0 and 15 mm.

7. The lead of claim 1, wherein the diameter of the needle is at most of 1.5 French (0.5 mm).

8. The lead of claim 1, wherein the outer surface of the needle is electrically insulated, at the exception of at least one naked area (72), located in the active free part (40), forming said at least one stimulation electrode.

9. The lead of claim 8, wherein the active free part (40) comprising said at least one naked part extends over at most 6 mm from the free distal end of the needle.

10. The lead of claim 8, comprising a plurality of distinct naked areas (72) extended in succession along the active free part and separated by non-naked insulating intermediate areas (74).

11. The lead of claim 8, wherein the total surface of the naked area(s) of the active free part is at most of 6 mm².

12. The lead of claim 1, wherein, at its proximal part, the needle is continued by a rod (56) to which it is fastened and which is welded to the inner conductor (46).

13. The lead of claim 1, wherein the anchoring helical screw (50) is electrically inactive.

14. The lead of claim 1, wherein the tubular body (48) is electrically inactive.
